# EUROPEAN PATENT APPLICATION

(11) **EP 4 772 104 A1**
(43) Date of publication of application: **08.07.2026**
(21) Application number: 24859679.3
(22) Date of filing: 26.08.2024
(51) Int. Cl.: A61B 1/045

(54) **COMPUTER PROGRAM, LEARNED MODEL GENERATION METHOD, IMAGE PROCESSING METHOD, AND IMAGE PROCESSING DEVICE**

(30) Priority: 28.08.2023 JP 2023138136
(71) Applicant: JMEES INC., Kashiwa-shi, Chiba 277-0882 (JP); National Cancer Center, Tokyo 104-0045 (JP)
(72) Inventor: MATSUZAKI, Hiroki, Kashiwa-shi, Chiba 277-0882 (JP); KOUNO, Atsushi, Kashiwa-shi, Chiba 277-0882 (JP); TAKESHITA, Nobuyoshi, Kashiwa-shi, Chiba 277-0882 (JP); FUJITA, Takeo, Tokyo 104-0045 (JP); SATO, Kazuma, Tokyo 104-0045 (JP)
(74) Representative: Cabinet Beaumont
(86) International application number: PCT/JP2024/030152
(87) International publication number: WO 2025/047646

(57) **Abstract**

Provided are a computer program, a trained model generation method, an image processing method, and an image processing device for easily obtaining a risk of nerve injury.

A computer executes a process for supporting endoscopic surgery according to a computer program. The computer program causes the computer to acquire an endoscopic image obtained by imaging an inside of a body using an endoscope and to output information indicating a risk of injury to a nerve inside the body, based on the acquired endoscopic image.

## Description

### Technical Field

The present invention relates to a computer program, a trained model generation method, an image processing method, and an image processing device used in endoscopic surgery.

### Background Art

As one method of surgery, there is endoscopic surgery in which an endoscope or a surgical instrument is inserted into a body to perform surgery. A technique has been developed that displays an endoscopic image obtained by imaging the inside of the body using an endoscope such that a doctor can operate a surgical instrument while viewing the endoscopic image in order to support endoscopic surgery. Patent Literature 1 discloses an example of a technology for supporting endoscopic surgery.

One of the complications that can occur after surgery is nerve paralysis. For example, recurrent laryngeal nerve paralysis may occur after surgery of the neck or the esophagus. Nerve paralysis occurs due to nerve injury such as overstretching. During endoscopic surgery, nerve injury may occur due to operations performed with surgical instruments. In order to check the risk of nerve injury, Intraoperative Nerve Monitoring (IONM) is used that performs the application of electrical stimulation to the nerve and the observation of a nerve action potential, using a tube with electrodes attached to the nerve.

### Citation List

### Patent Literature:

Patent Literature 1: WO2021/152784

### Summary

### Technical Problems

When IONM is used, it is not possible to use muscle relaxants since the muscle relaxants significantly reduce nerve responses. In addition, the attachment of the tube with electrodes to the nerve and the observation of the nerve action potential impose a burden on the surgeon.

The present invention has been made in view of the above circumstances, and an object of the present invention is to provide a computer program, a trained model generation method, an image processing method, and an image processing device for easily obtaining a risk of nerve injury.

### Solution to Problems

A computer program according to one aspect of the present invention is characterized by causing a computer to execute a process of: acquiring an endoscopic image obtained by imaging an inside of a body using an endoscope; and outputting information indicating a risk of injury to a nerve inside the body based on the acquired endoscopic images.

The computer program according to one aspect of the present invention is characterized by causing the computer to further execute a process of: inputting the acquired endoscopic image to a first trained model, which outputs risk information related to a risk of nerve injury when the endoscopic image is input, and acquiring the risk information output by the first trained model; and outputting information indicating the risk of nerve injury related to the acquired endoscopic image according to the acquired risk information.

In the computer program according to one aspect of the present invention, it is characterized in that the risk includes a risk of overstretching of the nerve, overflexion of the nerve, or thermal injury to the nerve caused by a surgical instrument.

The computer program according to one aspect of the present invention is characterized by causing the computer to further execute a process of: outputting the information indicating the risk in a format corresponding to a level of the risk.

The computer program according to one aspect of the present invention is characterized by causing the computer to further execute a process of: inputting the acquired endoscopic image to a second trained model, which detects a recurrent laryngeal nerve in an endoscopic image when the endoscopic image is input, and acquiring a detection result of the recurrent laryngeal nerve output by the second trained model; and coloring the recurrent laryngeal nerve in the acquired endoscopic image according to the detection result and outputting the endoscopic image.

The computer program according to one aspect of the present invention is characterized by causing the computer to further execute a process of: adjusting a display aspect of the recurrent laryngeal nerve in the endoscopic image according to the information indicating the risk.

The computer program according to one aspect of the present invention is characterized by causing the computer to further execute a process of: individually performing switching between the output of the information indicating the risk and stopping the output of the information indicating the risk and switching between the coloring of the recurrent laryngeal nerve in the endoscopic image and stopping the coloring of the recurrent laryngeal nerve in the endoscopic image.

The computer program according to one aspect of the present invention is characterized by causing the computer to further execute a process of: adjusting an operation of a surgical instrument according to the information indicating the risk.

The computer program according to one aspect of the present invention is characterized by causing the computer to further execute a process of: acquiring positions of the nerve and a surgical instrument in the acquired endoscopic image; acquiring depth positions of the nerve and the surgical instrument in the acquired endoscopic image; determining whether or not the surgical instrument is in a heat generating state; determining the risk of thermal injury to the nerve based on the positions and depth positions of the nerve and the surgical instrument in the acquired endoscopic image and a state of the surgical instrument; and outputting information indicating the determined risk.

The computer program according to one aspect of the present invention is characterized by causing the computer to further execute a process of: acquiring a position of the nerve in the acquired endoscopic image; acquiring a depth position of the nerve in the acquired endoscopic image; setting a plurality of points included in the nerve in the acquired endoscopic image; measuring a change in a distance between the points over time or a change in an angle formed between a plurality of straight lines connecting the points, based on the positions and depth positions of the plurality of points; determining a risk of overstretching or overflexion of the nerve based on the change in the distance over time or the change in the angle over time; and outputting information indicating the determined risk.

The computer program according to one aspect of the present invention is characterized by causing the computer to further execute a process of: outputting information indicating a point at which the risk of overstretching or overflexion is high among the plurality of points.

A trained model generation method according to one aspect of the present invention is characterized by comprising: acquiring training data including an endoscopic image obtained by imaging an inside of a body using an endoscope and risk information related to a risk of injury to a nerve inside the imaged body; and generating a trained model, which outputs the risk information when the endoscopic image is input, through learning using the training data.

The trained model generation method according to one aspect of the present invention is characterized by further comprising: acquiring the endoscopic image; acquiring the risk information using intraoperative nerve monitoring; and associating the risk information with the endoscopic image acquired when the risk information has been acquired to generate the training data.

An image processing method according to one aspect of the present invention is characterized by comprising: acquiring an endoscopic image obtained by imaging an inside of a body using an endoscope; and outputting information indicating a risk of injury to a nerve inside the body, based on the acquired endoscopic image.

An image processing device according to one aspect of the present invention is characterized by comprising: a calculation unit, wherein the calculation unit acquires an endoscopic image obtained by imaging an inside of a body using an endoscope and outputs information indicating a risk of injury to a nerve inside the body, based on the acquired endoscopic image.

In an aspect of the present invention, when the endoscope is used, the information indicating the risk of nerve injury is output based on the endoscopic image obtained by imaging the inside of the body of the patient. The user can perform endoscopic surgery while checking the output risk. In addition, the burden on the surgeon is reduced as compared to when IONM is used, and muscle relaxants can be used.

In an aspect of the present invention, the risk information is acquired using the first trained model that outputs the risk information related to the risk of nerve injury when the endoscopic image is input, and the information indicating the risk of nerve injury is output according to the risk information. The first trained model is generated by learning using the training data including the endoscopic image and the risk information. The risk of nerve injury during endoscopic surgery is related to the shape of the nerve and the positional relationship between the nerve and the surgical instrument. Therefore, the risk information related to the risk of nerve injury can be obtained based on the endoscopic image showing the shape of the nerve and the positional relationship between the nerve and the surgical instrument.

In an aspect of the present invention, the risk of nerve injury includes the risk of overstretching of the nerve, overflexion of the nerve, or thermal injury to the nerve caused by the surgical instrument. Possible nerve injury includes the overstretching of the nerve, the overflexion of the nerve, or thermal injury to the nerve, and a risk that these injuries will occur can be obtained.

In an aspect of the present invention, the information indicating the risk of nerve injury is output in a format corresponding to the level of the risk. The user can recognize the level of the risk of nerve injury.

In an aspect of the present invention, the detection result of the recurrent laryngeal nerve is obtained using the second trained model that outputs the detection result of the recurrent laryngeal nerve when the endoscopic image is input, and the endoscopic image, in which the recurrent laryngeal nerve has been colored, is output. When viewing the endoscopic image, the user can check the position of the recurrent laryngeal nerve.

In an aspect of the present invention, the display aspect of the recurrent laryngeal nerve is adjusted according to the risk of nerve injury. The user can view the recurrent laryngeal nerve and recognize the risk of nerve injury according to a change in the display aspect of the recurrent laryngeal nerve such as a change in color.

In an aspect of the present invention, the switching between the output of the information indicating the risk of nerve injury and stopping the output of the information indicating the risk of nerve injury is performed, and the switching between the coloring of the recurrent laryngeal nerve and stopping the coloring of the recurrent laryngeal nerve is performed. It is possible to output the endoscopic image in a state in which the information indicating the risk of nerve injury is not output or in a state in which the recurrent laryngeal nerve is not emphasized.

In an aspect of the present invention, the operation of the surgical instrument is adjusted according to the information indicating the risk of nerve injury. It is possible to control the operation of the surgical instrument such that the risk of nerve injury is reduced and to work for prevention of nerve injury.

In an aspect of the present invention, the positions and depth positions of the nerve and the surgical instrument in the endoscopic images are estimated, it is determined whether or not the surgical instrument is in the heat generating state, and the risk of thermal injury to the nerve is determined. When the surgical instrument is close to the nerve and is in the heat generating state, it can be determined that the risk of thermal injury to the nerve is high.

In an aspect of the present invention, a plurality of points included in the nerve in the acquired endoscopic image are set, the change in the distance between the points or the change in the angle formed between the straight lines connecting the points over time is measured, and the risk of overstretching or overflexion of the nerve is determined based on the measured change over time. When an increment in the distance between two points or the amount of change in the angle between two straight lines connecting any point and each of adjacent points is large, it can be determined that the risk of overstretching or overflexion of the nerve is high. In addition, information indicating the point at which the risk of overstretching or overflexion is high is output, which enables the user to recognize the location where the risk of overstretching or overflexion is high in the recurrent laryngeal nerve.

### Advantageous Effects of Invention

The present invention exhibits excellent effects such as the ability to easily obtain the risk of nerve injury.

### Brief Description of Drawings

FIG. 1 is a block diagram showing an example of a configuration of a surgical system for performing endoscopic surgery.
FIG. 2 is a block diagram showing an example of an internal configuration of an image processing device according to Embodiment 1.
FIG. 3 is a conceptual diagram showing an example of functions of a nerve detection model.
FIG. 4 is a conceptual diagram showing an example of functions of a risk detection model.
FIG. 5 is a flowchart showing an example of a procedure of a process according to Embodiment 1 which is executed by the image processing device when an endoscope is used.
FIG. 6 is a schematic view showing an example of an endoscopic image.
FIG. 7 is a schematic view showing an example of an endoscopic image in which a recurrent laryngeal nerve has been emphasized.
FIG. 8 is a schematic view showing an example of the output of information indicating the risk of nerve injury.
FIG. 9 is a schematic view showing an example of the output of the information indicating the risk of nerve injury.
FIG. 10 is a schematic view showing an example of the output of the information indicating the risk of nerve injury.
FIG. 11 is a block diagram showing an example of an internal functional configuration of a learning device.
FIG. 12 is a flowchart showing an example of a procedure of a process by which the learning device generates the risk detection model.
FIG. 13 is a block diagram showing an example of an internal configuration of an image processing device according to Embodiment 2.
FIG. 14 is a conceptual diagram showing an example of functions of an instrument detection model.
FIG. 15 is a conceptual diagram showing an example of functions of a depth estimation model.
FIG. 16 is a flowchart showing an example of a procedure of a process according to Embodiment 2 which is executed by the image processing device when an endoscope is used.
FIG. 17 is a flowchart showing an example of the procedure of the process according to Embodiment 2 which is executed by the image processing device when the endoscope is used.
FIG. 18 is a schematic view showing an example in which a plurality of points included in the recurrent laryngeal nerve in the endoscopic image are set.
FIG. 19 is a schematic view showing an example of a notification image displayed to be superimposed on the endoscopic image.

### Description of Embodiments

Hereinafter, the present invention will be described in detail with reference to the drawings showing embodiments of the invention.

### <Embodiment 1>

In endoscopic surgery, an endoscope and a surgical instrument are inserted into a body, and the inside of the body is imaged by the endoscope. A surgeon operates the surgical instrument to perform surgery while viewing the captured endoscopic image of the inside of the body. An example of the endoscope is a thoracoscope or a laparoscope that is inserted into the body of a patient through an opening formed in the body. An example of the surgical instrument is forceps or an electrosurgical scalpel. During endoscopic surgery, nerve injury may occur due to operations with the surgical instrument. When nerve injury occurs, nerve paralysis can occur. For example, after endoscopic surgery of the neck or the esophagus, recurrent laryngeal nerve paralysis may occur as a complication. In this embodiment, when the endoscope is used, information indicating the risk of nerve injury is output according to the situation to ensure the safety of the endoscopic surgery.

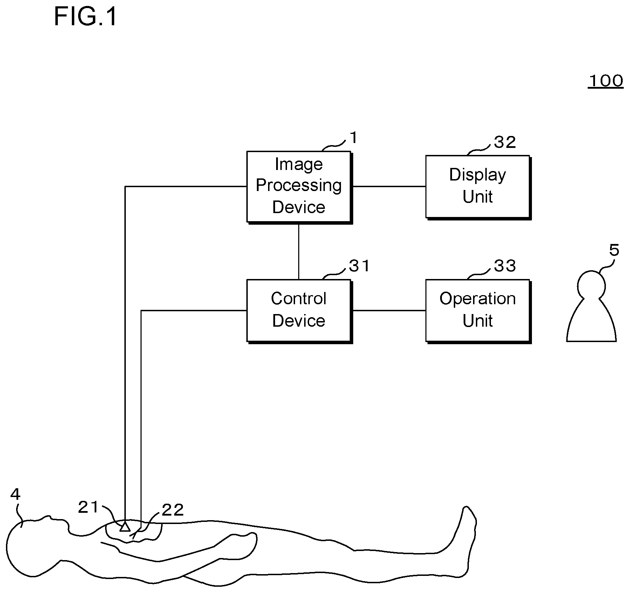

FIG. 1 is a block diagram showing an example of a configuration of a surgical system 100 for performing endoscopic surgery. Endoscopic surgery is performed on a patient 4. An endoscope 21 and a surgical instrument 22 are inserted into the body of the patient 4. The endoscope 21 images the inside of the body of the patient 4 and generates an endoscopic image. The endoscopic image is a color image. A plurality of surgical instruments 22, such as forceps or electrosurgical scalpels, may be inserted into the body of the patient 4. For example, the surgical instrument 22 is connected to a drive mechanism (not shown), such as a robot arm, for driving the surgical instrument 22 and is operated inside the body of the patient 4.

The surgical instrument 22 is connected to a control device 31 that controls the operation of the surgical instrument 22. The control device 31 is configured using a computer including a calculation unit that executes calculations for controlling the operation of the surgical instrument 22 and a memory. For example, the control device 31 controls the operation of the drive mechanism for driving the surgical instrument 22, thereby controlling the operation of the surgical instrument 22. An operation unit 33 that is operated by a user 5, such as a surgeon, is connected to the control device 31. The operation unit 33 is configured using a touch panel, a pointing device, a button, a lever, or the like. The user 5 operates the operation unit 33, and the control device 31 controls the operation of the surgical instrument 22 according to the operation received by the operation unit 33.

The endoscope 21 is connected to an image processing device 1. The image processing device 1 executes an image processing method for outputting information indicating the risk of nerve injury based on the endoscopic image. The image processing device 1 is connected to the control device 31. A display unit 32 that displays an image is connected to the image processing device 1. The display unit 32 is, for example, a liquid crystal display or an electroluminescent display (EL display). The endoscope 21 inputs the generated endoscopic image to the image processing device 1, and the image processing device 1 acquires the endoscopic image input from the endoscope 21 and displays the endoscopic image on the display unit 32. The endoscopic image obtained by imaging the inside of the body of the patient 4 is displayed on the display unit 32. The user 5 views the displayed endoscopic image to check the inside of the body of the patient 4. The user 5 operates the operation unit 33 to operate the surgical instrument 22 while viewing the endoscopic image, thereby performing endoscopic surgery.

In this embodiment, an example will be mainly described in which endoscopic surgery is performed on the neck or esophagus of the patient 4 using the surgical system 100 and information indicating the risk of injury to the recurrent laryngeal nerve is output. The endoscope 21 and the surgical instrument 22 are inserted into the body of the patient 4. The endoscope 21 images the inside of the body. The surgical instrument 22 is used to perform operations, such as tissue incision, suturing, or removal of lesions, inside the body.

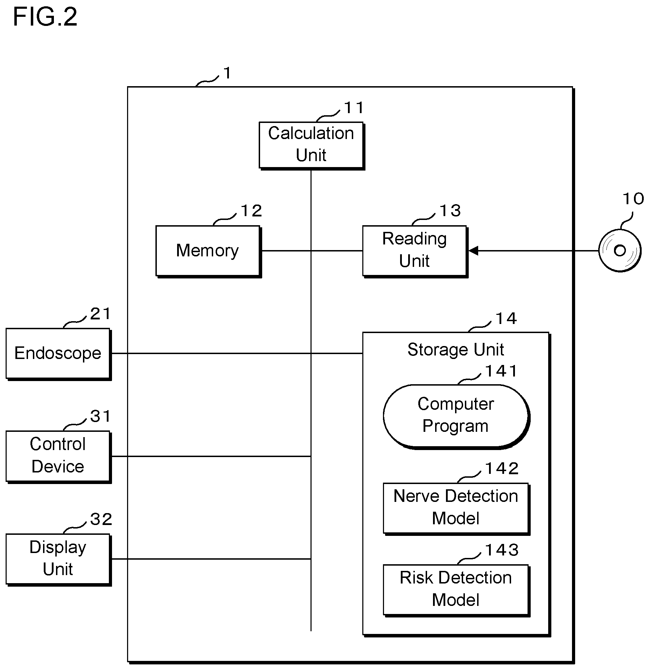

FIG. 2 is a block diagram showing an example of an internal configuration of the image processing device 1 according to Embodiment 1. The image processing device 1 is configured using a computer such as a personal computer or a server device. The image processing device 1 executes a computer operation method. The image processing device 1 includes a calculation unit 11, a memory 12, a reading unit 13, and a storage unit 14. The calculation unit 11 is configured using, for example, a central processing unit (CPU), a graphics processing unit (GPU), or a multi-core CPU. The calculation unit 11 may be configured using a quantum computer. The memory 12 stores temporary data generated in association with calculations. The memory 12 is, for example, a random access memory (RAM). The reading unit 13 reads information from a recording medium 10 such as an optical disk or a portable memory. The storage unit 14 is nonvolatile and is, for example, a hard disk or a non-volatile semiconductor memory.

The endoscope 21, the control device 31, and the display unit 32 are connected to the image processing device 1. For example, the endoscope 21, the control device 31, and the display unit 32 are connected to the calculation unit 11 via a communication interface (not shown). The image processing device 1 receives the endoscopic image from the endoscope 21 as input. The calculation unit 11 transmits a control signal to the control device 31. In addition, the calculation unit 11 transmits image data to the display unit 32 such that an image based on the image data is displayed on the display unit 32.

The calculation unit 11 causes the reading unit 13 to read a computer program 141 recorded on the recording medium 10 and stores the read computer program 141 in the storage unit 14. The calculation unit 11 executes a process for implementing the functions of the image processing device 1 according to the computer program 141. The computer program 141 may be a computer program product. The computer program 141 may be stored in the storage unit 14 in advance or may be downloaded from the outside of the image processing device 1. In this case, the image processing device 1 may not include the reading unit 13.

The computer program 141 can be deployed so as to be executed on a single computer or on a plurality of computers that are disposed at one site or that are distributed across a plurality of sites and connected to each other by a communication network. That is, the image processing device 1 may be configured by a plurality of computers, and the computer program 141 may be executed on the plurality of computers that are connected via the communication network. The image processing device 1 may be configured using a cloud server.

The computer program 141 can be deployed to be executed on a single computer or on a plurality of computers that are disposed at one site or that are distributed across a plurality of sites and connected to each other by a communication network. That is, the image processing device 1 may be configured by a plurality of computers, and the computer program 141 may be executed on a plurality of computers that are connected via the communication network. The image processing device 1 may be configured using a cloud server. The image processing device 1 and the control device 31 may be integrated into one device.

The image processing device 1 includes a nerve detection model 142 that, when the endoscopic image obtained by imaging the inside of the body of the patient 4 is input, outputs a detection result of the recurrent laryngeal nerve included in the endoscopic image. The nerve detection model 142 is a trained model that has been trained in advance so as to output the detection result of the recurrent laryngeal nerve when the endoscopic image is input. The nerve detection model 142 corresponds to a second trained model.

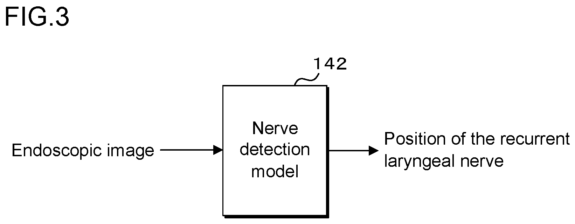

FIG. 3 is a conceptual diagram showing an example of the functions of the nerve detection model 142. The endoscopic image obtained by imaging the inside of the body of the patient 4 is input to the nerve detection model 142. The nerve detection model 142 has been trained in advance so as to output the position of the recurrent laryngeal nerve in the endoscopic image when the endoscopic image is input. The nerve detection model 142 is a trained model that performs segmentation. For example, the nerve detection model 142 is implemented using U-net or You Only Look Once (YOLO). Alternatively, the nerve detection model 142 may be implemented using Convolutional Neural Network (CNN). Other trained models may be used as the nerve detection model 142.

For example, the nerve detection model 142 outputs information indicating whether or not each pixel in the endoscopic image is a pixel corresponding to the recurrent laryngeal nerve, thereby outputting the position of the recurrent laryngeal nerve. The nerve detection model 142 may output information indicating the range of pixels corresponding to the recurrent laryngeal nerve among the pixels included in the endoscopic image. The nerve detection model 142 may output an image obtained by extracting the recurrent laryngeal nerve from the endoscopic image. In this way, the nerve detection model 142 outputs the detection result of the recurrent laryngeal nerve. The nerve detection model 142 outputs the detection result of the recurrent laryngeal nerve according to the endoscopic image, which makes it possible to detect the recurrent laryngeal nerve using the nerve detection model 142.

The calculation unit 11 executes information processing according to the computer program 141 to implement the nerve detection model 142. The storage unit 14 stores data necessary to implement the nerve detection model 142. A method for training the nerve detection model 142 will be described below.

Further, the image processing device 1 includes a risk detection model 143 that, when the endoscopic image obtained by imaging the inside of the body of the patient 4 is input, outputs risk information related to the risk that the nerve, which is located in the body and is present in the range captured by the endoscope 21, will be injured by the surgical instrument 22. The risk detection model 143 is a trained model that has been trained in advance so as to output the risk information when the endoscopic image is input. The risk detection model 143 corresponds to a first trained model.

The nerve injury is, for example, nerve overstretching, nerve overflexion, or thermal injury to the nerve. The overstretching or overflexion of the nerve may occur when the nerve is pulled or deformed during operations with the surgical instrument 22. Therefore, the risk of overstretching or overflexion of the nerve is related to the shape of the nerve. The thermal injury to the nerve can occur when the heated surgical instrument 22, such as an electrosurgical scalpel, approaches the nerve and heat is transferred from the surgical instrument 22 to the nerve. Therefore, the risk of thermal injury to the nerve is related to the positional relationship between the nerve and the surgical instrument 22. The shape of the nerve and the positional relationship between the nerve and the surgical instrument 22 can be obtained from the endoscopic image obtained by imaging the inside of the body with the endoscope 21. Therefore, the risk information related to the risk of nerve injury can be obtained based on the endoscopic image.

The risk information is information in which the risk of nerve injury is represented at a plurality of levels. More specifically, the risk information indicates the probability that the nerve included in the endoscopic image will be normal or the probability that the nerve will be injured. Alternatively, the risk information may indicate the degree of nerve injury that will occur. For example, the risk information may indicate the probability of nerve injury or the degree of nerve injury in three levels of safe, caution, and warning. For example, the risk information indicates the probability of nerve injury or the degree of nerve injury using a continuous value or a plurality of discrete values. The risk information may include information related to the risk of each of overstretching of the nerve, overflexion of the nerve, and thermal injury to the nerve caused by the surgical instrument 22.

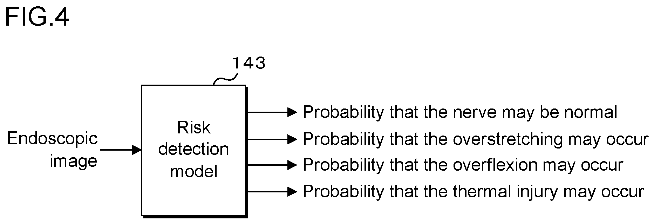

FIG. 4 is a conceptual diagram showing an example of the functions of the risk detection model 143. The endoscopic image obtained by imaging the inside of the body of the patient 4 is input to the risk detection model 143. The risk detection model 143 has been trained in advance so as to output, as the risk information, the probability that the nerve may be normal, the probability that the overstretching of the nerve may occur, the probability that the overflexion of the nerve may occur, and the probability that the thermal injury to the nerve may occur when the endoscopic image is input. The risk detection model 143 may be configured to output risk information including the degree of nerve overstretching, nerve overflexion, and thermal injury to the nerve that will occur, instead of the probability of nerve overstretching, nerve overflexion, and thermal injury to the nerve.

For example, the risk detection model 143 is implemented using CNN. Other trained models including Transformer may be used as the nerve detection model 142. The calculation unit 11 executes image processing according to the computer program 141 to implement the risk detection model 143. The storage unit 14 stores data necessary to implement the risk detection model 143. A method for training the risk detection model 143 will be described below.

The nerve detection model 142 or the risk detection model 143 may be configured using hardware. For example, the nerve detection model 142 or the risk detection model 143 may be configured by hardware including a processor and a memory for storing necessary programs and data. The nerve detection model 142 or the risk detection model 143 may be implemented using a quantum computer. Alternatively, the nerve detection model 142 or the risk detection model 143 may be provided outside the image processing device 1, and the image processing device 1 may be configured to execute processes using the external nerve detection model 142 or the external risk detection model 143. For example, the nerve detection model 142 or the risk detection model 143 may be implemented using the cloud.

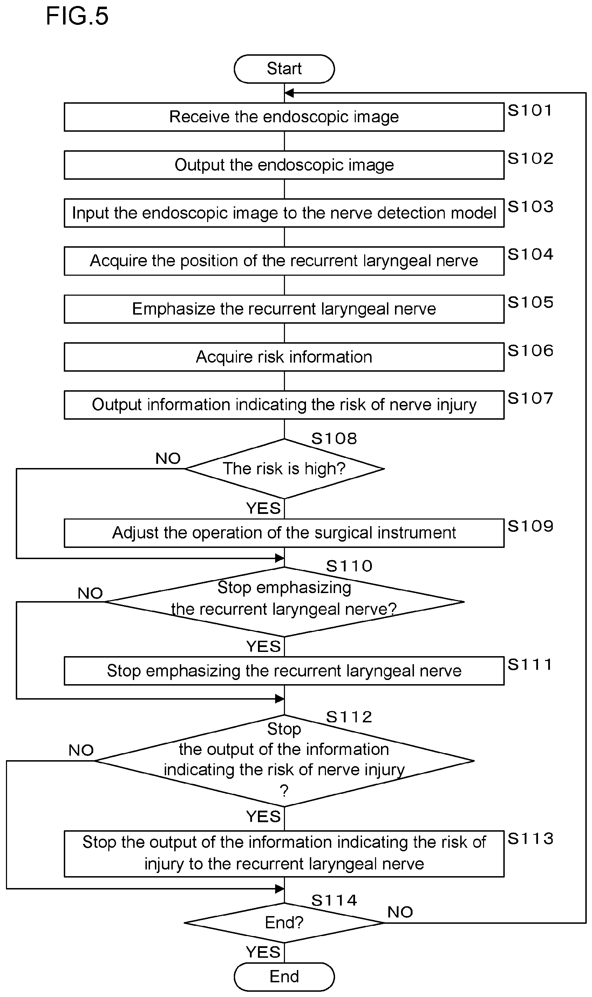

A process performed by the surgical system 100 when the endoscope 21 is used will be described. FIG. 5 is a flowchart showing an example of the procedure of the process according to Embodiment 1 which is executed by the image processing device 1 when the endoscope 21 is used. Hereinafter, a step will be abbreviated as S. The calculation unit 11 executes information processing according to the computer program 141 such that the image processing device 1 executes the following process.

An opening is formed in the body of the patient 4, and the endoscope 21 and the surgical instrument 22 are inserted into the body of the patient 4. A plurality of surgical instruments 22 may be inserted into the body of the patient 4. In this embodiment, the endoscope 21 and the surgical instrument 22 are inserted into the neck of the patient 4 or near the esophagus. The endoscope 21 images the inside of the body of the patient 4 to generate an endoscopic image and inputs the generated endoscopic image to the image processing device 1. The image processing device 1 receives the endoscopic image from the endoscope 21 as input and acquires the endoscopic image (S101). In S101, the calculation unit 11 stores the input endoscopic image in the storage unit 14 and reads the endoscopic image from the storage unit 14 to the memory 12, thereby acquiring the endoscopic image. The image processing device 1 outputs the acquired endoscopic image (S102). In S102, the calculation unit 11 displays the endoscopic image on the display unit 32.

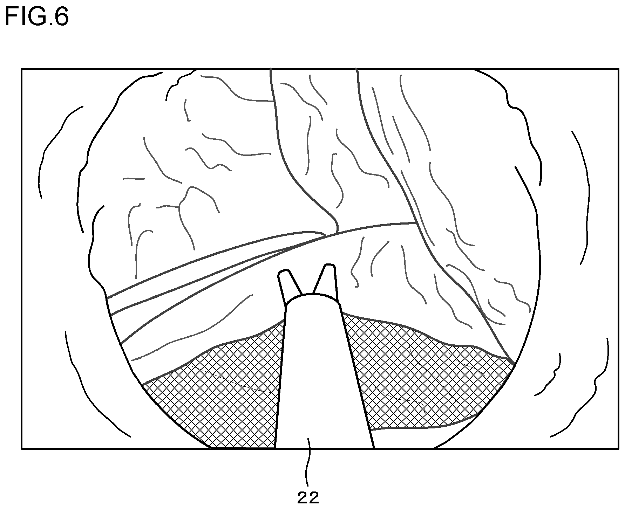

FIG. 6 is a schematic view showing an example of the endoscopic image. The displayed endoscopic image is a color image. The endoscopic image shows tissues present inside the body of the patient 4 and the surgical instrument 22. The user 5 views the endoscopic image displayed on the display unit 32 to observe the inside of the body of the patient 4. The user 5 operates the operation unit 33 while viewing the endoscopic image. The control device 31 controls the operation of the surgical instrument 22 according to the operation of the user 5 received by the operation unit 33. An operation, such as tissue incision, is performed with the surgical instrument 22 to perform endoscopic surgery. The image processing device 1 performs information processing in parallel with the execution of the endoscopic surgery to support the execution of the endoscopic surgery.

The image processing device 1 inputs the endoscopic image to the nerve detection model 142 (S103). In S103, the calculation unit 11 inputs the endoscopic image acquired in S101 to the nerve detection model 142. The nerve detection model 142 performs calculation in response to the input of the endoscopic image and outputs the position of the recurrent laryngeal nerve in the endoscopic image. The image processing device 1 acquires the position of the recurrent laryngeal nerve (S104). In S104, the calculation unit 11 acquires the position of the recurrent laryngeal nerve output by the nerve detection model 142.

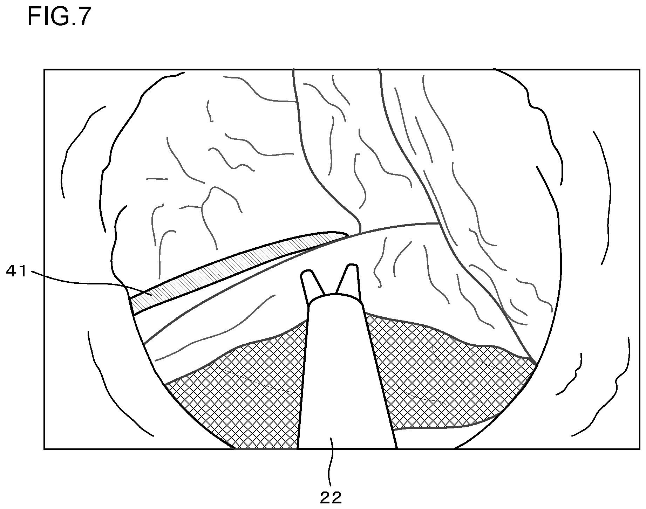

Then, the image processing device 1 emphasizes the recurrent laryngeal nerve in the endoscopic image to be output (S105). In S105, the calculation unit 11 generates an image obtained by emphasizing the recurrent laryngeal nerve in the endoscopic image and displays the generated image on the display unit 32. FIG. 7 is a schematic view showing an example of the endoscopic image in which the recurrent laryngeal nerve has been emphasized. The calculation unit 11 colors the recurrent laryngeal nerve 41 in the endoscopic image, based on the acquired position of the recurrent laryngeal nerve, to generate an image obtained by emphasizing the recurrent laryngeal nerve 41 in the endoscopic image. At this time, the calculation unit 11 colors the recurrent laryngeal nerve 41 in a color that is easy to see. For example, since the tissues inside the body shown in the endoscopic image are mainly red, the calculation unit 11 colors the recurrent laryngeal nerve 41 in blue. The calculation unit 11 colors the recurrent laryngeal nerve 41 and then displays the endoscopic image on the display unit 32.

When the endoscopic image in which the recurrent laryngeal nerve 41 has been emphasized is displayed, the user 5 viewing the endoscopic image can recognize the recurrent laryngeal nerve 41 included in the endoscopic image. The user 5 operates the operation unit 33 to control the operation of the surgical instrument 22 according to the position of the recurrent laryngeal nerve 41. For example, an operation using the surgical instrument 22 is performed while avoiding the recurrent laryngeal nerve 41. The image processing device 1 may emphasize the recurrent laryngeal nerve 41 in the endoscopic image using a method other than coloring. For example, the calculation unit 11 may emphasize the position of the recurrent laryngeal nerve 41 by adding an arrow or a bounding box indicating the position of the recurrent laryngeal nerve 41 to the endoscopic image or by depicting the recurrent laryngeal nerve 41 in white in the endoscopic image.

Then, the image processing device 1 acquires risk information related to the risk of nerve injury based on the endoscopic image (S106). In S106, the calculation unit 11 inputs the endoscopic image to the risk detection model 143. In this embodiment, the nerve is the recurrent laryngeal nerve. The risk detection model 143 performs calculation in response to the input of the endoscopic image and outputs the risk information. For example, as shown in FIG. 4, the risk detection model 143 outputs the risk information including the probability that the nerve will be normal, the probability that nerve overstretching will occur, the probability that nerve overflexion will occur, and the probability that thermal injury to the nerve will occur. The calculation unit 11 acquires the risk information output by the risk detection model 143.

In addition, an aspect may be adopted in which the image processing device 1 generates the risk information on a rule basis, without using the risk detection model 143, thereby acquiring the risk information. In this aspect, the calculation unit 11 generates the risk information according to a predetermined rule. The predetermined rule is included in the computer program 141. For example, the calculation unit 11 repeatedly acquires the endoscopic image and the position of the recurrent laryngeal nerve, determines the shape of the nerve each time it acquires the endoscopic image and the position of the recurrent laryngeal nerve, and generates the risk information at a plurality of levels according to the degree of deformation of the shape of the nerve from its original state. For example, when the rate of change in the size of the nerve or the magnitude of change in the angle of the nerve over the course of surgery exceeds a predetermined threshold value, the calculation unit 11 generates risk information indicating that the probability of nerve overstretching or nerve overflexion is high.

For example, the calculation unit 11 determines the position of the surgical instrument 22 in the endoscopic image, determines the distance between the surgical instrument 22 and the nerve, and generates the risk information at a plurality of levels according to the distance between the surgical instrument 22 and the nerve. For example, when the distance between the surgical instrument 22, which is an electrosurgical scalpel, and the nerve is less than a predetermined threshold value, the calculation unit 11 generates risk information indicating that the probability of thermal injury to the nerve is high. In the generated risk information, each of the degrees of nerve overstretching, nerve overflexion, and thermal injury to the nerve that have occurred may be represented at a plurality of levels.

An aspect may be adopted in which the image processing device 1 uses both the method of using the risk detection model 143 and the method of generating the risk information on the rule basis. In this aspect, the calculation unit 11 acquires final risk information, based on the risk information acquired using the risk detection model 143 and the risk information generated on the rule basis. For example, the calculation unit 11 averages the risk information acquired using the risk detection model 143 and the risk information generated on the rule basis to acquire the final risk information. For example, the calculation unit 11 adopts risk information indicating a higher risk of nerve injury, of the risk information acquired using the risk detection model 143 and the risk information generated on the rule basis, thereby acquiring the final risk information.

The calculation unit 11 may select one of the risk information acquired using the risk detection model 143 and the risk information generated on the rule basis and acquire the selected risk information. For example, which risk information is to be selected is preset, and the calculation unit 11 selects one of the risk information items according to the setting. For example, the user 5 operates the operation unit 33 to input a selection instruction to the image processing device 1, and the calculation unit 11 selects one of the risk information items according to the input instruction. Alternatively, the calculation unit 11 may individually select one of the information acquired using the risk detection model 143 and the information generated on the rule basis for information indicating the risk of each of nerve overstretching, nerve overflexion, and thermal injury to the nerve.

Then, the image processing device 1 outputs information indicating the risk of nerve injury according to the risk information (S107). In S107, the calculation unit 11 adds the information indicating the position of the recurrent laryngeal nerve and the information indicating the risk of nerve injury according to the risk information to the endoscopic image to generate an image, and displays the generated image on the display unit 32. The calculation unit 11 displays, on the display unit 32, an image including information indicating that, as the probability of nerve injury or the degree of nerve injury indicated by the risk information is higher, the risk of nerve injury is higher. For example, an image including an icon or a warning message indicating that the risk of nerve injury is high is displayed.

The calculation unit 11 adds the information indicating the risk of nerve injury to the endoscopic image in a plurality of forms according to a plurality of levels of the risk of injury based on the risk information. For example, a plurality of different icons are predefined according to a plurality of levels indicated by the risk information, and the calculation unit 11 adds an icon corresponding to the level of the risk of injury indicated by the risk information to the endoscopic image, thereby outputting the risk of nerve injury. For example, a plurality of warning messages are predefined according to a plurality of levels, and the calculation unit 11 adds a warning message corresponding to the level of the risk of injury indicated by the risk information to the endoscopic image. The warning message is displayed on the display unit 32 to be superimposed on the endoscopic image, thereby warning of the risk of nerve injury. For example, the calculation unit 11 adds a risk level bar or a safety level bar, which indicates the level of risk or safety by the length of the bar, to the endoscopic image according to the level of the risk of injury indicated by the risk information. The user 5 viewing the endoscopic image can recognize the level of the risk of nerve injury.

The calculation unit 11 may change the color of the endoscopic image displayed on the display unit 32 to output the risk of nerve injury. For example, the calculation unit 11 changes the color of all or a part of the endoscopic image. For example, the calculation unit 11 changes the color of the frame of the endoscopic image. The calculation unit 11 may change the color of the entire endoscopic image, the color of a part of the endoscopic image, or the color of the frame of the endoscopic image according to the level of the risk of injury based on the risk information. The calculation unit 11 may change the display aspect of the recurrent laryngeal nerve 41 in the endoscopic image to output the risk of nerve injury. For example, the calculation unit 11 may change the color of the recurrent laryngeal nerve 41 according to the level of the risk of injury based on the risk information. For example, when the risk is high, the calculation unit 11 may blink the recurrent laryngeal nerve 41. The user 5 can recognize the risk of nerve injury.

The calculation unit 11 may output information indicating the risk of nerve overstretching, nerve overflexion, and thermal injury to the nerve. For example, the calculation unit 11 outputs information indicating the risk of the most severe injury among nerve overstretching, nerve overflexion, and thermal injury to the nerve whose risk is indicated by the risk information. The calculation unit 11 may output information indicating the risk of each of nerve overstretching, nerve overflexion, and thermal injury to the nerve.

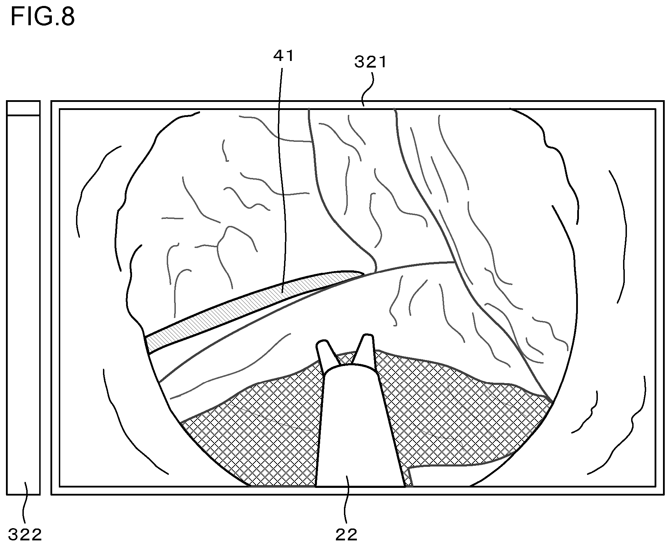

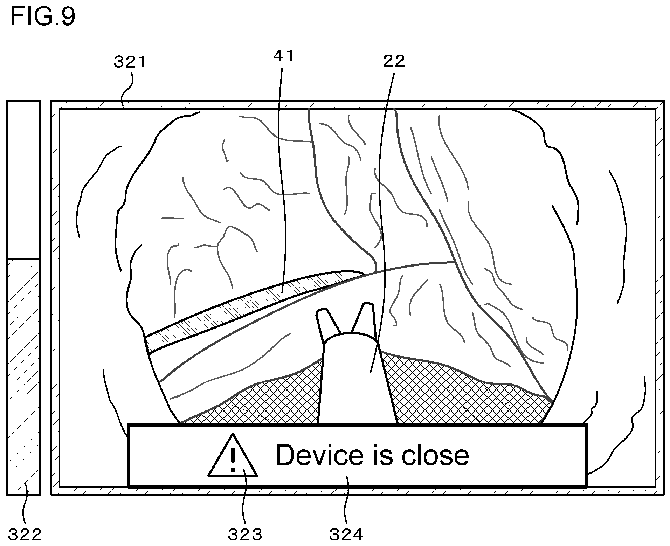

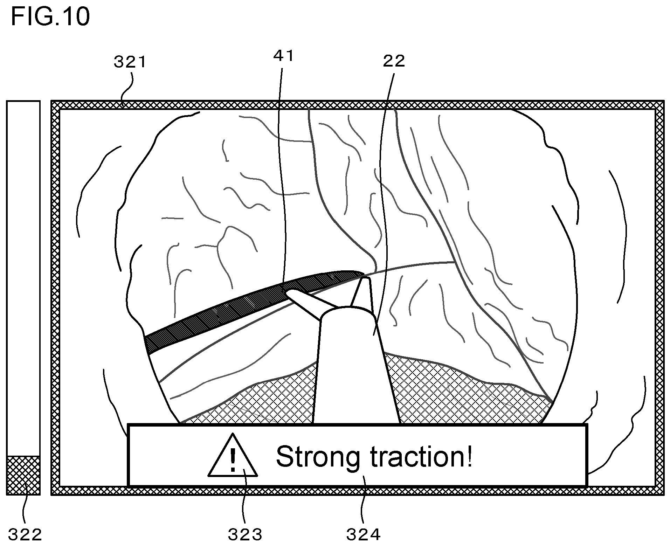

FIGS. 8 to 10 are schematic views showing an example of the output of the information indicating the risk of nerve injury. FIG. 8 shows an example of the output when the surgical instrument 22 is far from the recurrent laryngeal nerve 41 and the risk of injury to the recurrent laryngeal nerve 41 is relatively low. FIG. 9 shows an example of the output when the distance between the recurrent laryngeal nerve 41 and the surgical instrument 22 is short and the risk is higher. FIG. 10 shows an example of the output when the surgical instrument 22 is in contact with the recurrent laryngeal nerve 41 and the risk is even higher.

A frame 321 is added to the endoscopic image, and a safety level bar 322 that indicates the level of safety by the length of the bar is displayed. The calculation unit 11 determines the length of the safety level bar 322 according to the level of the risk of injury indicated by the risk information. Specifically, the calculation unit 11 increases the level of safety to increase the length of the safety level bar 322 as the level of the risk decreases and decreases the level of safety to decrease the length of the safety level bar 322 as the level of the risk increases. In the example shown in FIG. 8, since the risk is relatively low, the safety level bar 322 is long, which indicates that the level of safety is relatively high. In addition, the frame 321 and the safety level bar 322 are colored to show that the risk is low. For example, the colors of the frame 321 and the safety level bar 322 are green.

In the example shown in FIG. 9, an icon 323 indicating a higher risk of nerve injury and a warning message 324 stating "Device is close", which means that the distance between the recurrent laryngeal nerve 41 and the surgical instrument 22 is too short, are displayed to be superimposed on the endoscopic image. When the surgical instrument 22 is close to the recurrent laryngeal nerve 41, the possibility that the surgical instrument 22 will act on the recurrent laryngeal nerve 41 is high, and the risk of injury is high. The safety level bar 322 is shortened, which indicates a low level of safety, that is, a high risk. Further, the frame 321 and the safety level bar 322 are colored to show a higher risk. For example, the colors of the frame 321 and the safety level bar 322 are yellow.

In the example shown in FIG. 10, an icon 323 indicating a high risk of nerve injury and a warning message 324 stating "Strong traction!", which means that the surgical instrument 22 exerts a strong traction force on the recurrent laryngeal nerve 41, are displayed to be superimposed on the endoscopic image. When the recurrent laryngeal nerve 41 is subjected to traction and the traction force is strong, the risk of overstretching is very high. The safety level bar 322 is further shortened, which indicates that the level of safety is further reduced and the level of risk is further increased. Furthermore, the recurrent laryngeal nerve 41, the frame 321, and the safety level bar 322 are colored to show that the risk is further increased. For example, the colors of the frame 321 and the safety level bar 322 are red.

The user 5 viewing the output information shown in FIGS. 9 and 10 can recognize the risk of nerve injury, adjust the operation of the operation unit 33 to reduce the risk, and perform endoscopic surgery while preventing nerve injury. The image processing device 1 may cause the frame 321 to blink, thereby outputting information indicating the risk of nerve injury. The image processing device 1 may further change the icon 323 according to the level of the risk. An aspect may be adopted in which, when a plurality of surgical instruments 22 are inserted into the body, the image processing device 1 acquires risk information for each of the surgical instruments 22 and outputs the safety level bar 322 corresponding to a plurality of risk information items.

The image processing device 1 may output a warning message 324 corresponding to the level of the risk of nerve overstretching, nerve overflexion, or thermal injury to the nerve. For example, the warning message 324, such as "Nerve stretching detected", "Nerve stretching **%", or "Strong traction!", is displayed according to the level of the risk of nerve overstretching. For example, the warning message 324, such as "Nerve flexion detected", "Nerve flexion ** degrees", or "Flexion angle excessive!" is displayed according to the level of the risk of nerve overflexion. For example, the warning message 324, such as "Device is close", "Device is in contact", or "Risk of thermal injury to nerve! ", is displayed according to the level of the risk of thermal injury to the nerve. An aspect may be adopted in which the image processing device 1 includes a sound generation unit, such as a speaker, and outputs a warning sound corresponding to the level of the risk of nerve injury.

In S107, when the risk of nerve injury indicated by the risk information is low, the image processing device 1 may output information indicating that the risk is low or may not output the information indicating the risk. For example, when the level of the risk of nerve injury indicated by the risk information does not reach a predetermined level, the calculation unit 11 may display, on the display unit 32, an image obtained by adding the information indicating that the risk is low to the endoscopic image, or may not add the information indicating the risk to the endoscopic image.

The image processing device 1 determines whether or not the risk of nerve injury is high (S108). In S108, the calculation unit 11 determines the risk of nerve injury according to the risk information and determines whether or not the risk is high. For example, the calculation unit 11 determines that the risk is high when the level of the risk of injury indicated by the risk information exceeds a predetermined level.

When the risk of nerve injury is high (S108: YES), the image processing device 1 adjusts the operation of the surgical instrument 22 so as to reduce the risk of nerve injury (S109). In S109, the calculation unit 11 transmits a control signal for adjusting the operation of the surgical instrument 22 to the control device 31. The control device 31 receives the control signal and adjusts the operation of the surgical instrument 22 according to the control signal. At this time, the calculation unit 11 transmits a control signal for reducing the risk of nerve injury. For example, the calculation unit 11 transmits a control signal for further separating the surgical instrument 22 from the recurrent laryngeal nerve 41, and the control device 31 controls the operation of the surgical instrument 22 according to the control signal such that the surgical instrument 22 is further separated from the recurrent laryngeal nerve 41.

This adjustment of the operation of the surgical instrument 22 according to the information indicating the risk of nerve injury makes it possible to control the operation of the surgical instrument 22 such that the risk of nerve injury is reduced. As a result, it is possible to prevent injury to the nerve of the patient 4. The image processing device 1 may execute the processes in S108 and S109 before S107.

After S109 is ended or when the risk of nerve injury is low (S108: NO), the image processing device 1 determines whether or not to stop emphasizing the recurrent laryngeal nerve (S110). When the user 5 operates the operation unit 33 to input an instruction to stop emphasizing the recurrent laryngeal nerve to the control device 31, the control device 31 inputs the instruction to stop emphasizing the recurrent laryngeal nerve to the image processing device 1. In S110, when the instruction to stop emphasizing the recurrent laryngeal nerve is input from the control device 31, the calculation unit 11 determines to stop emphasizing the recurrent laryngeal nerve. When the instruction to stop emphasizing the recurrent laryngeal nerve is not input, the calculation unit 11 determines not to stop emphasizing the recurrent laryngeal nerve.

When it is determined that emphasizing the recurrent laryngeal nerve is stopped (S110: YES), the image processing device 1 stops emphasizing the recurrent laryngeal nerve (S111). In S 111, the calculation unit 11 displays an endoscopic image, in which the recurrent laryngeal nerve has not been colored, on the display unit 32. When the recurrent laryngeal nerve is emphasized by a method other than the coloring, the calculation unit 11 displays the endoscopic image, in which the recurrent laryngeal nerve has not been emphasized, on the display unit 32. The image processing device 1 may execute the processes in S110 and S111 at other timings.

After S111 is ended or when it is determined that emphasizing the recurrent laryngeal nerve is not stopped (S110: NO), the image processing device 1 determines whether or not to stop the output of the information indicating the risk of nerve injury (S112). When the user 5 operates the operation unit 33 to input an instruction to stop the output of the information indicating the risk to the control device 31, the control device 31 inputs the instruction to stop the output of the information indicating the risk to the image processing device 1. In S112, when the instruction to stop the output of the information indicating the risk is input from the control device 31, the calculation unit 11 determines to stop the output of the information indicating the risk. When the instruction to stop the output of the information indicating the risk is not input, the calculation unit 11 determines not to stop the output of the information indicating the risk.

When it is determined that the output of the information indicating the risk of nerve injury is stopped (S112: YES), the image processing device 1 stops the output of the information indicating the risk (S113). In S113, the calculation unit 11 displays an endoscopic image, to which the information indicating the risk of nerve injury has not been added, on the display unit 32. The image processing device 1 may execute the processes in S112 and S113 at other timings.

Through Steps S110 to S113, the switching between emphasizing the recurrent laryngeal nerve and stopping the emphasis of the recurrent laryngeal nerve and the switching between outputting the information indicating the risk of nerve injury and stopping the output of the information indicating the risk of nerve injury are performed. When the emphasis of the recurrent laryngeal nerve or the output of the information indicating the risk of nerve injury are stopped, the endoscopic image, in which the recurrent laryngeal nerve has not been emphasized, or the endoscopic image, to which the information indicating the risk of nerve injury has not been added, is displayed on the display unit 32. The user 5 can check the endoscopic image in detail without being obstructed by the emphasized recurrent laryngeal nerve or without being obstructed by the information indicating the risk of nerve injury.

After S113 is ended or when it is determined that the output of the information indicating the risk of nerve injury is not stopped (S112: NO), the image processing device 1 determines whether or not to end the process of outputting the endoscopic image (S114). When the endoscopic surgery is ended, the control device 31 inputs information indicating the end of the endoscopic surgery to the image processing device 1. A configuration may be adopted in which, when the user 5 operates the operation unit 33 to input an instruction to end the endoscopic surgery to the control device 31, the control device 31 inputs information indicating the end of the endoscopic surgery to the image processing device 1. In S114, when the information indicating the end of the endoscopic surgery is input from the control device 31, the calculation unit 11 determines to end the process of outputting the endoscopic image. When the information indicating the end of the endoscopic surgery is not input, the calculation unit 11 determines not to end the process of outputting the endoscopic image.

When it is determined that the process of outputting the endoscopic image is not ended (S114: NO), the image processing device 1 returns the process to S101. The image processing device 1 repeats the processes in S101 to S114 in parallel with the execution of the endoscopic surgery. When it is determined that the process of outputting the endoscopic image is ended (S114: YES), the image processing device 1 ends the information processing.

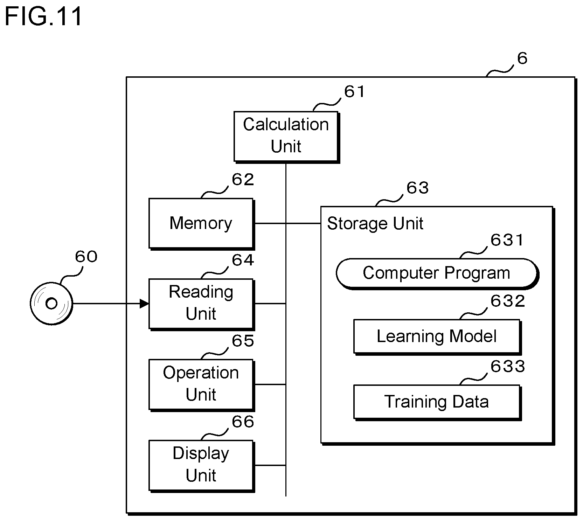

A method for training the nerve detection model 142 and the risk detection model 143 will be described. The nerve detection model 142 and the risk detection model 143 are trained by a learning device 6. FIG. 11 is a block diagram showing an example of an internal functional configuration of the learning device 6. The learning device 6 is a computer such as a server device or a personal computer. The learning device 6 executes a computer operation method. The learning device 6 includes a calculation unit 61, a memory 62, a storage unit 63, a reading unit 64, an operation unit 65, and a display unit 66. The calculation unit 61 is configured using, for example, a CPU, a GPU, or a multi-core CPU. The calculation unit 61 may also be configured using a quantum computer. The memory 62 stores temporary data generated in association with calculations. The memory 62 is, for example, a RAM. The reading unit 64 reads information from a recording medium 60 such as an optical disk or a portable memory. The storage unit 63 is non-volatile and is, for example, a hard disk or a non-volatile semiconductor memory.

The operation unit 65 receives an operation from the user 5 to receive the input of information. The operation unit 65 is, for example, a keyboard, a pointing device, or a touch panel. The display unit 66 displays an image. For example, the display unit 66 is a liquid crystal display or an EL display.

The calculation unit 61 causes the reading unit 64 to read a computer program 631 recorded on the recording medium 60 and stores the read computer program 631 in the storage unit 63. The calculation unit 61 executes a process for implementing the functions of the learning device 6 according to the computer program 631. The computer program 631 may be a computer program product. The computer program 631 may be stored in the storage unit 63 in advance or may be downloaded from the outside of the learning device 6. In this case, the learning device 6 may not include the reading unit 64.

The computer program 631 can be deployed to be executed on a single computer or on a plurality of computers that are disposed at one site or that are distributed across a plurality of sites and connected to each other by a communication network. That is, the learning device 6 may be configured by a plurality of computers, and the computer program 631 may be executed on the plurality of computers that are connected via the communication network. The learning device 6 may be configured using a cloud server.

The learning device 6 includes a learning model 632 that is the basis of the nerve detection model 142 or the risk detection model 143. The learning device 6 trains the learning model 632 to generate the nerve detection model 142 or the risk detection model 143. The calculation unit 61 executes a process according to the computer program 631 to implement the learning model 632. The learning model 632 has the same configuration as the nerve detection model 142 or the risk detection model 143. The learning model 632 may be configured using hardware.

The storage unit 63 stores training data 633 for training the learning model 632 to generate the nerve detection model 142 or the risk detection model 143. A data set in which an endoscopic image is associated with the position of the recurrent laryngeal nerve in the endoscopic image is recorded in the training data 633 for generating the nerve detection model 142. For example, a person checks the generated endoscopic image and finds the recurrent laryngeal nerve in the endoscopic image, and the position of the found recurrent laryngeal nerve is recorded in association with the endoscopic image. A plurality of data sets are recorded in the training data 633.

The learning device 6 trains the learning model 632 with the training data 633 to generate the nerve detection model 142. The calculation unit 61 inputs the endoscopic image recorded in the training data 633 to the learning model 632 that is the basis of the nerve detection model 142. The learning model 632 performs calculation in response to the input of the endoscopic image and outputs the position of the recurrent laryngeal nerve.

The calculation unit 61 adjusts parameters of calculation in the learning model 632 such that an error between the position of the recurrent laryngeal nerve output by the learning model 632 and the position of the recurrent laryngeal nerve associated with the input endoscopic image is reduced. That is, the calculation unit 61 adjusts the parameters such that the output position of the recurrent laryngeal nerve is close to the position of the recurrent laryngeal nerve associated with the input endoscopic image. For example, the calculation unit 61 adjusts the parameters using an error backpropagation method.

The calculation unit 61 repeats the process using the plurality of data sets recorded in the training data 633 to adjust the parameters of the learning model 632, thereby performing machine learning of the learning model 632. The calculation unit 61 adjusts the parameters of calculation in the learning model 632 in this way to generate the nerve detection model 142. The calculation unit 61 stores the final adjusted parameters in the storage unit 63.

The generated nerve detection model 142 is included in the image processing device 1. For example, the final adjusted parameters of the learning model 632 are input to the image processing device 1 and stored in the storage unit 14. The calculation unit 11 executes information processing using the stored parameters to implement the nerve detection model 142.

The learning device 6 executes a trained model generation method to generate the risk detection model 143. A data set, in which the endoscopic image is associated with the risk information related to the risk of injury to the nerve in the endoscopic image, is recorded in the training data 633 for generating the risk detection model 143. The risk information included in the training data 633 is acquired, for example, using Intraoperative Neurophysiological Monitoring (IONM).

In IONM, a tube with electrodes is attached, and electrical stimulation of the nerve and observation of the nerve action potential are performed. When the endoscope is used, IONM is executed, and the output of the endoscopic image and the output of the nerve action potential are performed. When an abnormality, such as loss, occurs in the nerve action potential observed by IONM, there is a possibility that nerve injury has occurred. The nerve action potential is acquired by the information processing device. When an abnormality occurs in the nerve action potential, the information processing device generates risk information corresponding to the abnormality that has occurred. For example, risk information, in which the probability of nerve injury or the degree of injury that has occurred is represented at a plurality of levels, is generated according to the abnormality in the nerve action potential. For example, risk information including the probability that the nerve will be normal, the probability that nerve overstretching will occur, the probability that nerve overflexion will occur, and the probability that thermal injury to the nerve will occur is generated according to the nerve action potential.

The information processing device stores the endoscopic image acquired when an abnormality occurs in the nerve action potential and the generated risk information in association with each other to generate a data set in which the endoscopic image and the risk information are associated with each other. The learning device 6 receives the generated data set as input and stores the training data 633, in which the input data set has been recorded, in the storage unit 63, thereby generating the training data 633. The data set included in the training data 633 may be generated by methods other than the method using IONM. For example, a person checks the endoscopic image and estimates risk information, and a data set in which the endoscopic image and the estimated risk information are associated with each other is recorded in the training data 633. A plurality of data sets are recorded in the training data 633.

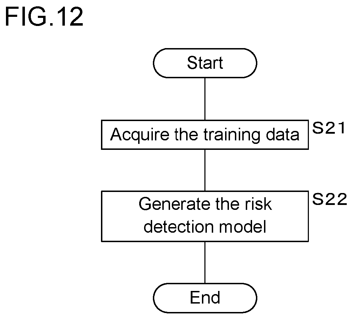

FIG. 12 is a flowchart showing an example of the procedure of the process by which the learning device 6 generates the risk detection model 143. The calculation unit 61 executes information processing according to the computer program 631 such that the learning device 6 executes the following process. By calculation unit 61 reading the training data 633 stored in the storage unit 63, and the learning device 6 acquires the training data 633 (S21). In S21, the learning device 6 may acquire the training data 633 by being input the training data 633 from the outside.

Then, the learning device 6 trains the learning model 632 with the training data 633 to generate the risk detection model 143 (S22). In S22, the calculation unit 61 inputs the endoscopic image recorded in the training data 633 to the learning model 632 that is the basis of the risk detection model 143. The learning model 632 performs calculation in response to the input of the endoscopic image and outputs risk information.

The calculation unit 61 adjusts parameters of calculation in the learning model 632 such that the error between the risk information output by the learning model 632 and the risk information associated with the input endoscopic image is reduced. That is, the calculation unit 61 adjusts the parameters such that the risk information associated with the input endoscopic image approximately matches the output risk information. For example, the calculation unit 61 adjusts the parameters using the error backpropagation method.

The calculation unit 61 repeats the process using a plurality of data sets recorded in the training data 633 to adjust the parameters of the learning model 632, thereby performing machine learning of the learning model 632. The calculation unit 61 adjusts the calculation parameters of the learning model 632 in this way to generate the risk detection model 143. The calculation unit 61 stores the final adjusted parameters in the storage unit 63. After S22 is ended, the learning device 6 ends the process.

The risk detection model 143 generated in S21 and S22 is included in the image processing device 1. For example, the final adjusted parameters of the learning model 632 are input to the image processing device 1 and stored in the memory unit 14. The calculation unit 11 executes information processing using the stored parameters to implement the risk detection model 143. In addition, the image processing device 1 may also have the function of the learning device 6 generating the nerve detection model 142 or the risk detection model 143. The image processing device 1 executes the processes in S101 to S114, using the generated nerve detection model 142 or risk detection model 143.

As described above in detail, in this embodiment, the image processing device 1 outputs the risk of nerve injury caused by the surgical instrument 22, based on the endoscopic image obtained by imaging the inside of the body of the patient 4. The information indicating the risk of nerve injury is displayed on the display unit 32, and the user 5 can perform endoscopic surgery while checking the risk of nerve injury. It is possible to perform endoscopic surgery while taking measures to prevent nerve injury, such as measures to stop the operation, when the risk of nerve injury is high.

In this embodiment, since the risk of nerve injury is obtained based on the endoscopic image, it is possible to detect that the risk of nerve injury is high before nerve injury actually occurs. Therefore, it is possible to perform endoscopic surgery while effectively preventing nerve injury. Since there is no need to attach the tube with electrodes to the nerve, the burden on the surgeon is reduced, as compared to when IONM is used. Further, in this embodiment, since there is no need to observe the nerve action potential, it is possible to use muscle relaxants that cannot be used in IONM. Therefore, it is possible to easily reduce the risk of nerve injury during endoscopic surgery. It is possible to perform endoscopic surgery according to the easily obtained risk while taking measures to prevent nerve injury, and the safety of endoscopic surgery is improved. Since muscle relaxants can be used, sudden patient movements (bucking) do not occur during surgery, which makes it possible to perform safe surgery in terms of anesthesia management. Furthermore, since the muscle relaxants can be used, flexibility in endoscopic surgery is improved.

When the risk of nerve injury is high, stress may be applied to the nerve even when actual nerve injury does not occur. Stress on the nerve may cause an adverse event such as numbness. When stress is applied to the recurrent laryngeal nerve, hoarseness or voice weakness may occur as an adverse event. In addition, as a result of stress on the recurrent laryngeal nerve, vocal cord movement may be reduced, and an adverse event, such as reduced swallowing function or aspiration pneumonia, may occur. In this embodiment, the risk of nerve injury is output to indicate that stress may be applied to the nerve. When the risk of nerve injury is high, the operation is stopped, which makes it possible to prevent adverse events caused by stress on the nerve.

In this embodiment, an example has been described in which the risk detection model 143 outputs the risk information including the information related to the risk of nerve overstretching, nerve overflexion, and thermal injury to the nerve. The information related to the risk of nerve overstretching, nerve overflexion, and thermal injury to the nerve may be obtained using separate trained models. For example, a trained model that outputs the probability of nerve overstretching when an endoscopic image is input, a trained model that outputs the probability of nerve overflexion when an endoscopic image is input, and a trained model that outputs the probability of thermal injury to the nerve when an endoscopic image is input may be used individually. In addition to these trained models, a trained model may be used that outputs the probability that the nerve will be normal when an endoscopic image is input.

In this embodiment, the risk detection model 143 outputs the risk information when the endoscopic image is input. However, an aspect may be adopted in which the risk detection model 143 outputs the risk information when the endoscopic image and the position of the nerve are input. In this aspect, the risk detection model 143 has been trained in advance so as to output the risk information when the endoscopic image and the position of the nerve are input.

In this aspect, the risk detection model 143 receives the endoscopic image obtained by imaging the inside of the body of the patient 4 and the information indicating the position of the nerve in the endoscopic image as input. Specifically, the information indicating the position of the recurrent laryngeal nerve acquired in S104 is input. For example, the information indicating the position of the nerve is information indicating pixels corresponding to the nerve among the pixels included in the endoscopic image. An image obtained by adding the information indicating the position of the nerve to the endoscopic image may be input to the risk detection model 143. The risk detection model 143 is generated by learning using training data in which the endoscopic image, the position of the nerve, and the risk information are associated with each other. Even in this aspect, it is possible to output the information indicating the risk of nerve injury, using the risk information output by the risk detection model 143.

### <Embodiment 2>

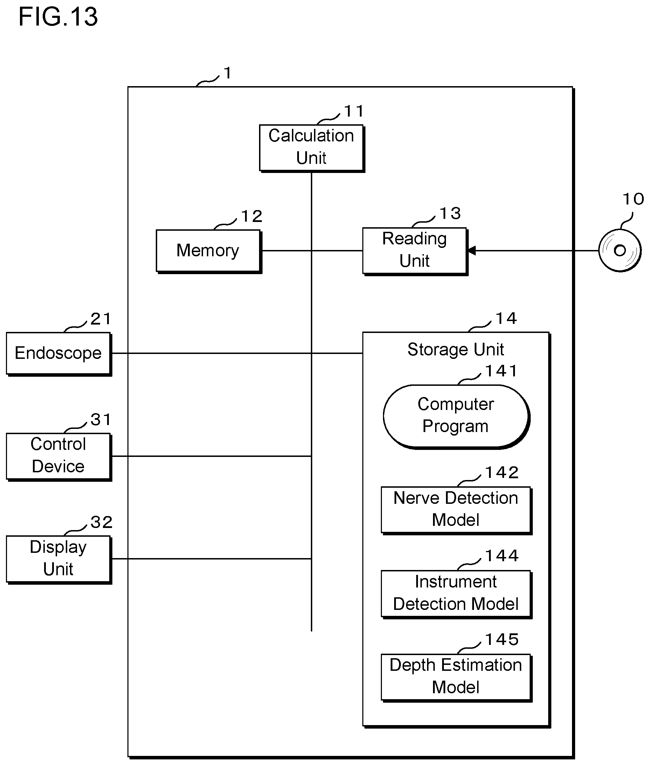

Embodiment 2 shows an aspect in which the risk of nerve injury is determined using a method different from the method using the risk detection model 143. A configuration of a surgical system 100 according to Embodiment 2 is the same as that in Embodiment 1. FIG. 13 is a block diagram showing an example of an internal configuration of an image processing device 1 according to Embodiment 2. The image processing device 1 includes a nerve detection model 142, as in Embodiment 1. In addition, the image processing device 1 includes an instrument detection model 144 that outputs a detection result of a surgical instrument included in an endoscopic image when the endoscopic image is input and a depth estimation model 145 that outputs the depth positions of the recurrent laryngeal nerve and the surgical instrument included in the endoscopic image. The other configurations of the image processing device 1 are the same as those in Embodiment 1.

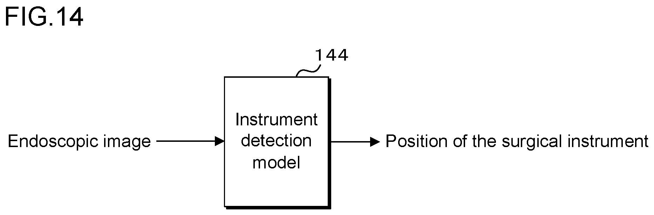

FIG. 14 is a conceptual diagram showing an example of the functions of the instrument detection model 144. The instrument detection model 144 receives an endoscopic image obtained by imaging the inside of the body of the patient 4 as input. The instrument detection model 144 is a trained model and has been trained in advance so as to output the position of the surgical instrument included in the endoscopic image when the endoscopic image is input. The output position of the surgical instrument is a position in the plane of the endoscopic image. The instrument detection model 144 is a trained model that performs segmentation. For example, the instrument detection model 144 is implemented using U-net or YOLO. Alternatively, the instrument detection model 144 may be implemented using CNN or may be implemented using other trained models.

For example, the instrument detection model 144 outputs information indicating whether or not each pixel in the endoscopic image is a pixel corresponding to the surgical instrument, thereby outputting the position of the surgical instrument in the endoscopic image. The instrument detection model 144 may output information indicating the range of pixels corresponding to the surgical instrument among the pixels included in the endoscopic image. The instrument detection model 144 may output an image obtained by extracting the surgical instrument from the endoscopic image. In this way, the instrument detection model 144 outputs the detection result of the surgical instrument.

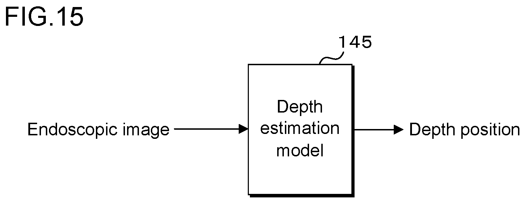

FIG. 15 is a conceptual diagram showing an example of the functions of the depth estimation model 145. The depth estimation model 145 receives the endoscopic image obtained by imaging the inside of the body of the patient 4 as input. The depth estimation model 145 is a trained model and has been trained in advance so as to output the depth position of each portion included in the endoscopic image when the endoscopic image is input. The depth position is a position in a depth direction orthogonal to the endoscopic image and is the position of each object shown in the endoscopic image in the depth direction. For example, the depth position is a distance from the endoscope 21 to each object shown in the endoscopic image or the relative position of the object shown in the endoscopic image in the depth direction.

For example, the depth estimation model 145 outputs information indicating the depth position of each pixel in the endoscopic image, thereby outputting the depth position of each portion included in the endoscopic image. The depth estimation model 145 outputs the depth position of each portion of the recurrent laryngeal nerve and the surgical instrument included in the endoscopic image, thereby outputting the depth positions of the recurrent laryngeal nerve and the surgical instrument.

The calculation unit 11 executes information processing according to the computer program 141 to implement the instrument detection model 144 and the depth estimation model 145. The storage unit 14 stores data necessary to implement the instrument detection model 144 and the depth estimation model 145. The instrument detection model 144 has been trained in advance using training data including data in which the endoscopic image is associated with the position of the surgical instrument in the endoscopic image. The depth estimation model 145 has been trained in advance using training data including data in which the endoscopic image is associated with the positions of the recurrent laryngeal nerve and the surgical instrument in the endoscopic image. For example, the instrument detection model 144 and the depth estimation model 145 have been trained using the training device 6.

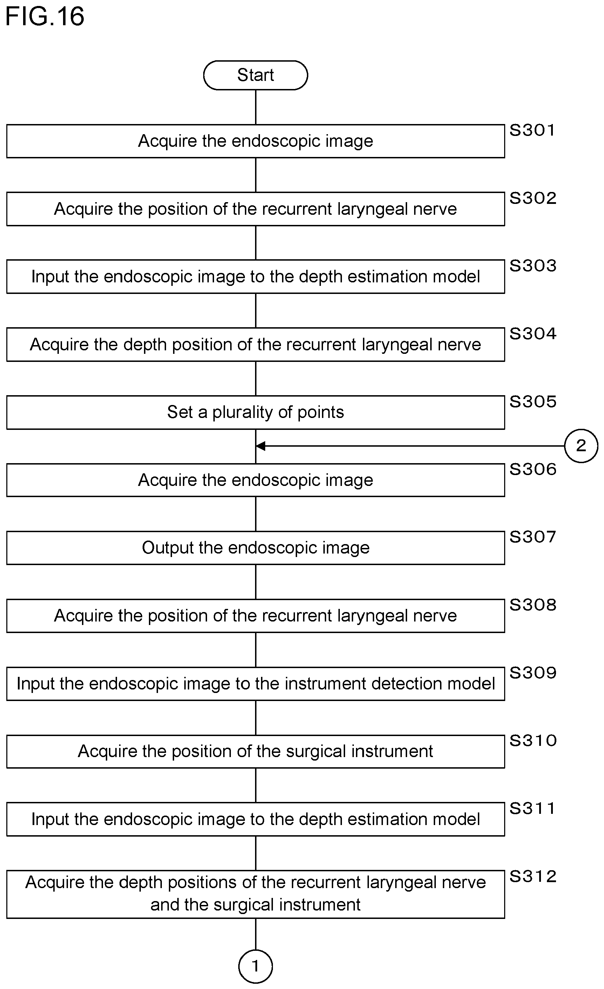

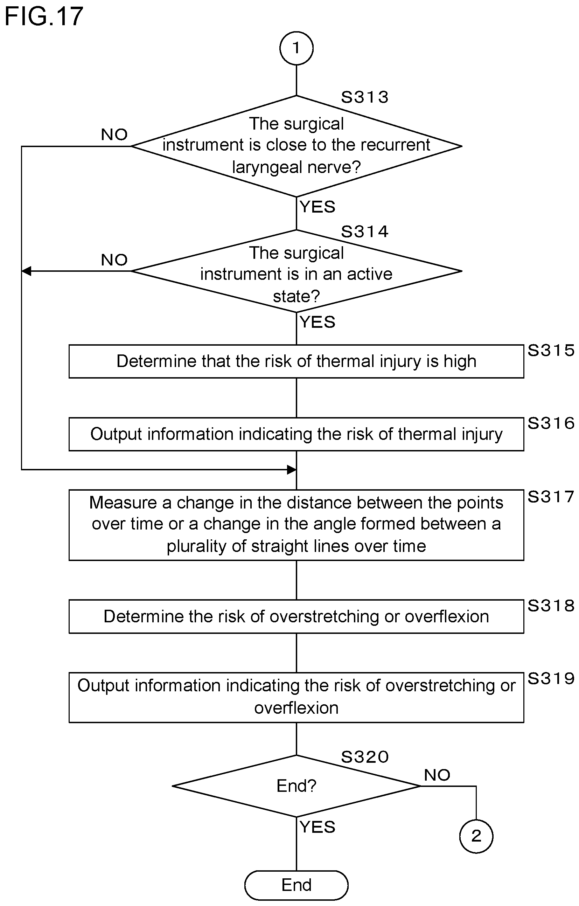

FIGS. 16 and 17 are flowcharts showing an example of a procedure of a process according to Embodiment 2 which is executed by the image processing device 1 when the endoscope 21 is used. The endoscope 21 and the surgical instrument 22 are inserted into the body of the patient 4, and the endoscope 21 images the inside of the body of the patient 4 to generate an endoscopic image. In Embodiment 2, the endoscope 21 and the surgical instrument 22 are inserted into the neck or near the esophagus of the patient 4 as in Embodiment 1. The image processing device 1 receives the endoscopic image from the endoscope 21 as input and acquires the endoscopic image (S301). Then, the image processing device 1 acquires the position of the recurrent laryngeal nerve (S302). In S302, the calculation unit 11 acquires the position of the recurrent laryngeal nerve using the nerve detection model 142 as in Embodiment 1. The acquired position of the recurrent laryngeal nerve is a position in the plane of the endoscopic image.

The image processing device 1 inputs the endoscopic image to the depth estimation model 145 (S303). In S303, the calculation unit 11 inputs the endoscopic image acquired in S301 to the depth estimation model 145. The depth estimation model 145 performs calculation in response to the input of the endoscopic image and outputs the depth position of each portion in the endoscopic image. The image processing device 1 acquires the depth position of the recurrent laryngeal nerve (S304). In S304, the calculation unit 11 acquires the depth position of the recurrent laryngeal nerve in the endoscopic image output by the depth estimation model 145.

The image processing device 1 sets a plurality of points included in the recurrent laryngeal nerve 41 in the endoscopic image (S305). In S305, the calculation unit 11 arranges the plurality of points in the recurrent laryngeal nerve along a longitudinal direction of the recurrent laryngeal nerve to associate the plurality of points with a plurality of portions of the recurrent laryngeal nerve. The calculation unit 11 sets the position and depth position of each portion of the recurrent laryngeal nerve associated with each point in the endoscopic image as the position and depth position of each point and stores the initial values of the position and depth position of each point in the memory unit 14.

In S305, the calculation unit 11 may dispose the plurality of points on a center line of the recurrent laryngeal nerve or may dispose the plurality of points along the contour of the recurrent laryngeal nerve. The calculation unit 11 may appropriately use both methods. For example, the calculation unit 11 arranges the plurality of points at equal intervals in the recurrent laryngeal nerve. The intervals between the points may be predetermined. The number of points to be disposed in the recurrent laryngeal nerve may be predetermined, and the calculation unit 11 may adjust the intervals between the points according to the length of the recurrent laryngeal nerve and the number of points. The intervals between the plurality of points may not be equal. The points may be disposed in a characteristic portion of the recurrent laryngeal nerve. The calculation unit 11 may set the plurality of points, using a trained model that performs key point detection according to the image. The position of the point may be designated by an instruction from the user 5.

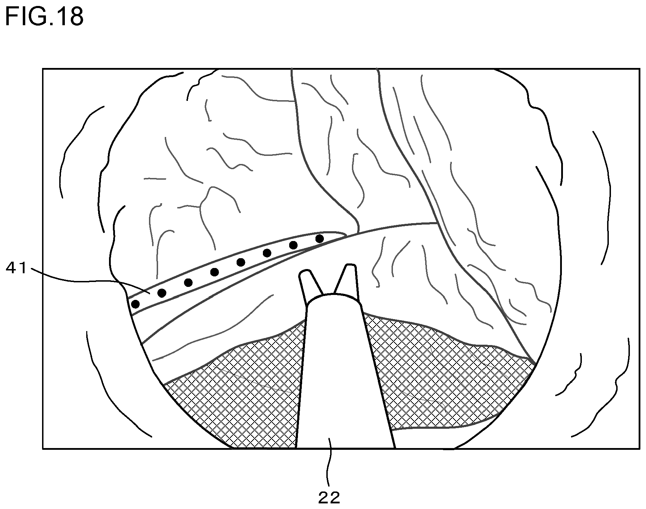

FIG. 18 is a schematic view showing an example in which a plurality of points included in the recurrent laryngeal nerve 41 in the endoscopic image are set. The plurality of points are represented by a plurality of black circles included in the recurrent laryngeal nerve 41 in the endoscopic image. The image processing device 1 may or may not display the endoscopic image including the plurality of points shown in FIG. 18 on the display unit 32.

The image processing device 1 receives the endoscopic image from the endoscope 21 as input, acquires the endoscopic image (S306), and outputs the acquired endoscopic image (S307). In S307, the calculation unit 11 displays the endoscopic image as shown in FIG. 6 on the display unit 32. Then, the image processing device 1 acquires the position of the recurrent laryngeal nerve (S308). In S308, the calculation unit 11 acquires the position of the recurrent laryngeal nerve in the endoscopic image using the nerve detection model 142 as in Embodiment 1. The acquired position of the recurrent laryngeal nerve is a position in the plane of the endoscopic image. In addition, the image processing device 1 emphasizes the recurrent laryngeal nerve in the endoscopic image to be output as in Embodiment 1.

The image processing device 1 inputs the endoscopic image to the instrument detection model 144 (S309). In S309, the calculation unit 11 inputs the endoscopic image acquired in S306 to the instrument detection model 144. The instrument detection model 144 performs calculation in response to the input of the endoscopic image and outputs the position of the surgical instrument 22 in the endoscopic image. The image processing device 1 acquires the position of the surgical instrument 22 (S310). In S310, the calculation unit 11 acquires the position of the surgical instrument 22 in the endoscopic image output by the instrument detection model 144.

Then, the image processing device 1 inputs the endoscopic image to the depth estimation model 145 (S311). In S311, the calculation unit 11 inputs the endoscopic image acquired in S306 to the depth estimation model 145. The depth estimation model 145 performs calculation in response to the input of the endoscopic image and outputs the depth position of each portion in the endoscopic image. The image processing device 1 acquires the depth positions of the recurrent laryngeal nerve and the surgical instrument 22 (S312). In S312, the calculation unit 11 acquires the depth positions of the recurrent laryngeal nerve and the surgical instrument 22 in the endoscopic image output by the depth estimation model 145. The image processing device 1 may acquire the depth positions of the recurrent laryngeal nerve and the surgical instrument 22 without using the depth estimation model 145. For example, the endoscope 21 may include a depth camera, and the image processing device 1 may acquire the depth positions of the recurrent laryngeal nerve and the surgical instrument 22 based on an imaging result of the depth camera.

Then, the image processing device 1 determines whether or not the surgical instrument 22 is close to the recurrent laryngeal nerve based on the positions and depth positions of the recurrent laryngeal nerve and the surgical instrument 22 (S313). In S313, the calculation unit 11 calculates the distance between the recurrent laryngeal nerve and the surgical instrument 22 based on the positions and depth positions of the recurrent laryngeal nerve and the surgical instrument 22 in the endoscopic image and determines whether or not the surgical instrument 22 is close to the recurrent laryngeal nerve based on the calculated distance. For example, the calculation unit 11 calculates the shortest distance between the recurrent laryngeal nerve and the surgical instrument 22 and determines that the surgical instrument 22 is close to the recurrent laryngeal nerve when the calculated shortest distance is less than a predetermined threshold value. When the calculated shortest distance is not less than the predetermined threshold value, the calculation unit 11 determines that the surgical instrument 22 is not close to the recurrent laryngeal nerve. The calculation unit 11 may calculate a distance between a specific portion of the surgical instrument 22 and the recurrent laryngeal nerve and perform the determination using the calculated distance.

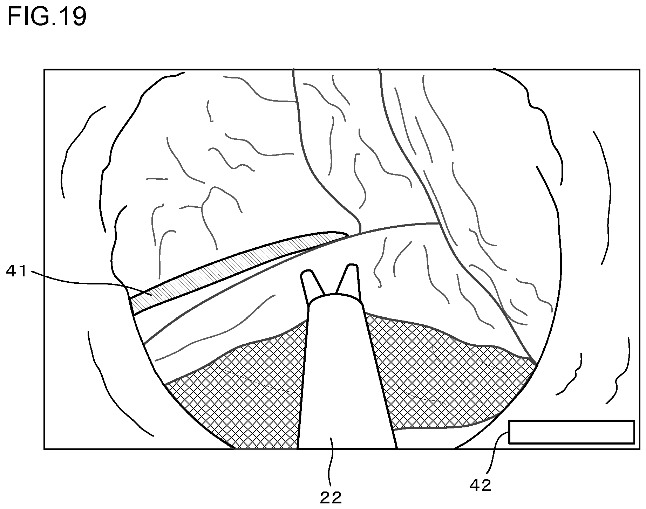

When the surgical instrument 22 is close to the recurrent laryngeal nerve (S313: YES), the image processing device 1 determines whether or not the surgical instrument 22 is in an active state in which the surgical instrument 22 generates heat (S314). The control device 31 controls the surgical instrument 22 such that the surgical instrument 22 switches between the active state and a non-heat-generating state. The image processing device 1 displays a notification image for notifying of the state of the surgical instrument 22 on the display unit 32 to be superimposed on the endoscopic image, based on a signal from the control device 31. FIG. 19 is a schematic view showing an example of a notification image 42 displayed to be superimposed on the endoscopic image. For example, the color of the notification image 42 changes depending on the state of the surgical instrument 22 to notify of the state of the surgical instrument 22. For example, the notification image 42 includes a message indicating the state of the surgical instrument 22. In S314, the calculation unit 11 determines the state of the surgical instrument 22 according to the notification image 42 displayed to be superimposed on the endoscopic image. The calculation unit 11 may be directly notified of the state of the surgical instrument 22 by the control device 31 to determine the state of the surgical instrument 22.

When the surgical instrument 22 is in the active state (S314: YES), the image processing device 1 determines that the risk of thermal injury to the recurrent laryngeal nerve is high (S315). In S315, the calculation unit 11 determines that the risk of immediate thermal injury to the recurrent laryngeal nerve is high when the surgical instrument 22 is in the active state. In addition, the calculation unit 11 may determine that the risk of thermal injury is high when a period of a state which the surgical instrument 22 is close to the recurrent laryngeal nerve and the surgical instrument 22 is in the active state exceeds a predetermined period. Furthermore, the calculation unit 11 may determine the level of the risk of thermal injury occurring in stages, according to the distance between the recurrent laryngeal nerve and the surgical instrument 22 and the period of the active state.

In addition, the image processing device 1 may perform the process of determining that the risk of injury to the recurrent laryngeal nerve is high when the surgical instrument 22 is close to the recurrent laryngeal nerve, regardless of whether or not the surgical instrument 22 is in the active state. Through this process, the image processing device 1 determines the risk that non-thermal injury, such as incision, will occur in the recurrent laryngeal nerve due to the surgical instrument 22.

The image processing device 1 outputs information indicating the risk of thermal injury to the recurrent laryngeal nerve (S316). In S316, the calculation unit 11 generates an image by adding information indicating the position of the recurrent laryngeal nerve and information indicating that the risk of thermal injury to the recurrent laryngeal nerve is high to the endoscopic image, and displays the generated image on the display unit 32. For example, the calculation unit 11 displays, on the display unit 32, an image including an icon or a warning message indicating that the risk of thermal injury to the recurrent laryngeal nerve is high. The calculation unit 11 may output the information indicating the risk of thermal injury to the recurrent laryngeal nerve, according to the level of the risk of thermal injury. For example, the calculation unit 11 may change the type of the icon, the content of the warning message, the color of the recurrent laryngeal nerve 41 in the endoscopic image, or the color of the endoscopic image, according to the level of the risk of thermal injury to the recurrent laryngeal nerve. The calculation unit 11 may add a risk level bar or a safety level bar to the endoscopic image, according to the level of the risk of thermal injury to the recurrent laryngeal nerve as in Embodiment 1.

When the surgical instrument 22 is not close to the recurrent laryngeal nerve (S313: NO), when the surgical instrument 22 is not in the active state (S314: NO), or after S316 is ended, the image processing device 1 measures a change in the distance between the points included in the recurrent laryngeal nerve 41 in the endoscopic image over time or a change in the angle formed between a plurality of straight lines connecting the points over time (S317). In S317, the calculation unit 11 specifies the positions and depth positions of a plurality of points at the present time according to the positions and depth positions of the recurrent laryngeal nerve and the surgical instrument 22 in the endoscopic image and stores the positions and depth positions of the plurality of points in the storage unit 14. In addition, the calculation unit 11 measures a change in the distance between the points over time or a change in the angle formed between a plurality of straight lines connecting the points over time, based on the stored positions and depth positions of the plurality of points at a plurality of time points. For example, the calculation unit 11 measures a change in the distance between two adjacent points over time. For example, the calculation unit 11 measures a change in the angle between two straight lines connecting each point and an adjacent point over time.

The image processing device 1 determines the risk of overstretching or overflexion of the recurrent laryngeal nerve, based on the change in the distance between the points over time or the change in the angle formed between a plurality of straight lines over time (S318). In S318, for example, when an increment in the distance between any two adjacent points from an initial value or from the previous time point exceeds a predetermined threshold value, the calculation unit 11 determines that the risk of overstretching of the recurrent laryngeal nerve is high. For example, when the amount of change in the angle between two straight lines connecting any point and its adjacent points exceeds a predetermined threshold value, the calculation unit 11 determines that the risk of overflexion of the recurrent laryngeal nerve is high.

When the distance between two adjacent points exceeds the threshold value at locations greater than a predetermined number of locations, the calculation unit 11 may determine that the risk of overstretching is high. When the amount of change in the angle formed between two straight lines exceeds the threshold value at locations greater than a predetermined number of locations, the calculation unit 11 may determine that the risk of overflexion is high. When the result of accumulating the amount of change in the distance between two points or the amount of change in the angle between two straight lines over the entire length of the recurrent laryngeal nerve exceeds a predetermined threshold value, the calculation unit 11 may determine that the risk of overstretching or overflexion of the recurrent laryngeal nerve is high.

The calculation unit 11 may determine the risk of overstretching or overflexion based on a time series of the distance between two points or a time series of the angle between two straight lines. For example, the calculation unit 11 may determine the risk of overstretching or overflexion based on an accumulated value of the time series of the distance or the angle or an integral of a graph showing the time series of the distance or the angle. The calculation unit 11 may perform the determination based on the time series of the distance between any two points or the time series of the angle between any two straight lines, or may perform the determination based on the accumulated value or integral of the time series of the distance or the angle over the entire length of the recurrent laryngeal nerve. The calculation unit 11 may perform the determination using a trained model that has been trained to output the risk of overstretching or overflexion of the recurrent laryngeal nerve when the time series of the distance between two points or the time series of the angle between two straight lines is input.

The calculation unit 11 may determine the level of the risk of overstretching or overflexion of the recurrent laryngeal nerve in stages. For example, the calculation unit 11 may determine the level of the risk of overstretching or overflexion, based on the increment in the distance between two points or the amount of change in the angle between two straight lines or based on the number of locations where the distance between two points exceeds the threshold value or the number of locations where the amount of change in the angle between two straight lines exceeds the threshold value. For example, the calculation unit 11 may determine the level of the risk of overstretching or overflexion, based on the accumulated value of the time series of the distance or the angle, or the integral of the graph showing the time series of the distance or the angle.

The image processing device 1 outputs information indicating the risk of overstretching or overflexion of the recurrent laryngeal nerve (S319). In S319, the calculation unit 11 outputs the information indicating the risk of overstretching or overflexion, according to the contrary result in S318. For example, the calculation unit 11 displays, on the display unit 32, an image including an icon or a warning message indicating that the risk of overstretching or overflexion of the recurrent laryngeal nerve is high. The calculation unit 11 may output the information indicating the risk of overstretching or overflexion, according to the level of the risk of overstretching or overflexion of the recurrent laryngeal nerve. For example, the calculation unit 11 may change the type of the icon, the content of the warning message, the color of the recurrent laryngeal nerve 41 in the endoscopic image, or the color of the endoscopic image, according to the level of the risk of overstretching or overflexion. The calculation unit 11 may add a risk level bar or a safety level bar to the endoscopic image, according to the level of the risk of overstretching or overflexion of the recurrent laryngeal nerve as in Embodiment 1.

In S319, when there is any location where the risk of overstretching or overflexion is high in the recurrent laryngeal nerve, the calculation unit 11 may output information indicating that the risk of overstretching or overflexion of the recurrent laryngeal nerve is high. The calculation unit 11 may display a point at which the risk of overstretching or overflexion is high among a plurality of points included in the recurrent laryngeal nerve on the display unit 32 to be superimposed on the recurrent laryngeal nerve 41 in the endoscopic image, thereby outputting information indicating the point at which the risk of overstretching or overflexion is high. The user 5 can recognize the location where the risk of overstretching or overflexion is high in the recurrent laryngeal nerve.

When it is determined in S318 that the risk of overstretching or overflexion of the recurrent laryngeal nerve is low, the image processing device 1 may output information indicating that the risk of overstretching or overflexion of the recurrent laryngeal nerve is low. At this time, the calculation unit 11 displays the information indicating that the risk of overstretching or overflexion is low on the display unit 32. When it is determined in S318 that the risk of overstretching or overflexion is low, the image processing device 1 may omit the process in S319.

The image processing device 1 determines whether or not to end the process (S320). In S320, when information indicating the end of the endoscopic surgery is input from the control device 31, the calculation unit 11 determines to end the process of outputting the endoscopic image. When the information indicating the end of the endoscopic surgery is not input, the calculation unit 11 determines not to end the process of outputting the endoscopic image. When it is determined that the process of outputting the endoscopic image is not ended (S320: NO), the image processing device 1 returns the process to S306. The image processing device 1 repeats the processes in S306 to S320 in parallel with the execution of the endoscopic surgery. When it is determined that the process of outputting the endoscopic image is ended (S320: YES), the image processing device 1 ends the information processing.

As described above, in Embodiment 2, the image processing device 1 determines the risk of nerve injury, using a method different from the method using the risk detection model 143, and outputs the information indicating the risk of nerve injury. The image processing device 1 can determine the risk of thermal injury, overstretching, or overflexion of the recurrent laryngeal nerve in more detail and notify the user 5 of the risk of thermal injury, overstretching, or overflexion, as compared to the method using the risk detection model 143.

In Embodiments 1 and 2, the example has been described in which the surgical system 100 is used to perform endoscopic surgery on the neck or esophagus of the patient 4. However, the surgical system 100 can also be used to perform endoscopic surgery on other parts of the patient 4. In Embodiments 1 and 2, the aspect has mainly been described in which the recurrent laryngeal nerve of the patient 4 is emphasized in the endoscopic image and the risk of injury to the recurrent laryngeal nerve is output. The image processing device 1 may be configured to emphasize nerves other than the recurrent laryngeal nerve and to output the risk of injury to nerves other than the recurrent laryngeal nerve when endoscopic surgery is performed on parts other than the neck or esophagus of the patient 4. For example, the nerve for which emphasis and injury risk output are performed may be a facial nerve, a vagus nerve, a hypogastric nerve, an obturator nerve, or a pelvic nerve.

In Embodiments 1 and 2, the endoscope, such as a thoracoscope or a laparoscope, that is inserted into the body of the patient through an opening formed in the body has been mainly described. However, an endoscope that is inserted into the body of the patient without forming any holes may be used, such as an endoscope that is inserted into the gastrointestinal tract. In Embodiments 1 and 2, nerve overstretching, nerve overflexion, and thermal injury to the nerve are given as specific examples of the nerve injury. However, the image processing device 1 may also be configured to output information indicating the risk of other types of injury. For example, the image processing device 1 may output information indicating the risk of nerve compression or crush as the risk of nerve injury. For example, the risk detection model 143 may be configured to output the probability of nerve compression or the probability of nerve crush as the risk information. Further, the surgical system 100 may be configured to separately include a display unit that displays an endoscopic image and a display unit that outputs the information indicating the risk of nerve injury. In Embodiments 1 and 2, the surgical instrument 22 is operated by a drive mechanism, such as a robot arm, and is operated using the operation unit 33. However, the surgical instrument 22 may be held and operated by the user 5 who is a surgeon.

The present invention is not limited to the above-described embodiments, and various modifications can be made within the scope of the claims. That is, embodiments obtained by combining technical means modified appropriately within the scope of the claims are also included in the technical scope of the present invention.

The matters described in each of the embodiments can be combined with each other. Further, the independent claims and the dependent claims described in the claims can be combined with each other in any and all combinations, regardless of the citation format. Furthermore, in the description of the claims, a format (multi-claim format) is used in which a claim cites two or more other claims, but the present invention is not limited thereto. In the description of the claims, a format may be used in which a multi-claim (multi-multi-claim) cites at least one other multi-claim.

### Reference Signs List

- 100: surgical system
- 1: image processing device
- 10: recording medium
- 11: calculation unit
- 14: storage unit
- 141: computer program
- 142: nerve detection model (second trained model)
- 143: risk detection model (first trained model)
- 21: endoscope
- 22: surgical instrument
- 31: control device
- 32: display unit
- 4: patient
- 5: user
- 6: learning device

## Claims

1. A computer program, **characterized by** causing a computer to execute a process of:
acquiring an endoscopic image obtained by imaging an inside of a body using an endoscope; and
outputting information indicating a risk of injury to a nerve inside the body based on the acquired endoscopic images.

2. The computer program according to claim 1, **characterized by** causing the computer to further execute a process of:
inputting the acquired endoscopic image to a first trained model, which outputs risk information related to a risk of nerve injury when the endoscopic image is input, and acquiring the risk information output by the first trained model; and
outputting information indicating the risk of nerve injury related to the acquired endoscopic image according to the acquired risk information.

3. The computer program according to claim 1, **characterized in that**
the risk includes a risk of overstretching of the nerve, overflexion of the nerve, or thermal injury to the nerve caused by a surgical instrument.

4. The computer program according to claim 1, **characterized by** causing the computer to further execute a process of:
outputting the information indicating the risk in a format corresponding to a level of the risk.

5. The computer program according to claim 1, **characterized by** causing the computer to further execute a process of:
inputting the acquired endoscopic image to a second trained model, which detects a recurrent laryngeal nerve in an endoscopic image when the endoscopic image is input, and acquiring a detection result of the recurrent laryngeal nerve output by the second trained model; and
coloring the recurrent laryngeal nerve in the acquired endoscopic image according to the detection result and outputting the endoscopic image.

6. The computer program according to claim 5, **characterized by** causing the computer to further execute a process of:
adjusting a display aspect of the recurrent laryngeal nerve in the endoscopic image according to the information indicating the risk.

7. The computer program according to claim 5, **characterized by** causing the computer to further execute a process of:
individually performing switching between the output of the information indicating the risk and stopping the output of the information indicating the risk and switching between the coloring of the recurrent laryngeal nerve in the endoscopic image and stopping the coloring of the recurrent laryngeal nerve in the endoscopic image.

8. The computer program according to claim 1, **characterized by** causing the computer to further execute a process of:
adjusting an operation of a surgical instrument according to the information indicating the risk.

9. The computer program according to claim 1, **characterized by** causing the computer to further execute a process of:
acquiring positions of the nerve and a surgical instrument in the acquired endoscopic image;
acquiring depth positions of the nerve and the surgical instrument in the acquired endoscopic image;
determining whether or not the surgical instrument is in a heat generating state;
determining the risk of thermal injury to the nerve based on the positions and depth positions of the nerve and the surgical instrument in the acquired endoscopic image and a state of the surgical instrument; and
outputting information indicating the determined risk.

10. The computer program according to claim 1, **characterized by** causing the computer to further execute a process of:
acquiring a position of the nerve in the acquired endoscopic image;
acquiring a depth position of the nerve in the acquired endoscopic image;
setting a plurality of points included in the nerve in the acquired endoscopic image;
measuring a change in a distance between the points over time or a change in an angle formed between a plurality of straight lines connecting the points, based on the positions and depth positions of the plurality of points;
determining a risk of overstretching or overflexion of the nerve based on the change in the distance over time or the change in the angle over time; and
outputting information indicating the determined risk.

11. The computer program according to claim 10, **characterized by** causing the computer to further execute a process of:
outputting information indicating a point at which the risk of overstretching or overflexion is high among the plurality of points.

12. A trained model generation method, **characterized by** comprising:
acquiring training data including an endoscopic image obtained by imaging an inside of a body using an endoscope and risk information related to a risk of injury to a nerve inside the imaged body; and
generating a trained model, which outputs the risk information when the endoscopic image is input, through learning using the training data.

13. The trained model generation method according to claim 12, **characterized by** further comprising:
acquiring the endoscopic image;
acquiring the risk information using intraoperative nerve monitoring; and
associating the risk information with the endoscopic image acquired when the risk information has been acquired to generate the training data.

14. An image processing method, **characterized by** comprising:
acquiring an endoscopic image obtained by imaging an inside of a body using an endoscope; and
outputting information indicating a risk of injury to a nerve inside the body, based on the acquired endoscopic image.

15. An image processing device, **characterized by** comprising:
a calculation unit,
wherein the calculation unit acquires an endoscopic image obtained by imaging an inside of a body using an endoscope and outputs information indicating a risk of injury to a nerve inside the body, based on the acquired endoscopic image.
